# EUROPEAN PATENT APPLICATION

(11) **EP 4 817 535 A1**
(43) Date of publication of application: **07.10.2026**
(21) Application number: 25315119.5
(22) Date of filing: 04.04.2025
(51) Int. Cl.: A61B 17/30, A61B 90/00

(54) **SURGICAL DEVICE FOR USE IN MICROSURGERY**

(71) Applicant: Carl Zeiss Meditec AG, 07745 Jena (DE); FCI S.A.S, 75015 Paris (FR); D.O.R.C. Dutch Ophthalmic Research Center (International) B.V., 3214 VN Zuidland (NL)
(72) Inventor: Meenink, Hildebert Christiaan Matthijs, 5656 AG Eindhoven (NL); Mitchell, Andrew, 5656 AG Eindhoven (NL); Lander, Frank, 3214 VN Zuidland (NL); Commiaux, Antoine, 25000 Besancon (FR); Abicht, Felix Johannes, 73447 Oberkochen (DE)
(74) Representative: DeltaPatents B.V.

(57) **Abstract**

A surgical device (1) is provided for use in microsurgery, comprising an outer tube (20) comprising a distal end, an instrument insert (30) extending axially from the distal end of the outer tube, wherein the instrument insert comprises at least two working ends (40, 50), wherein the outer tube is configured to slidably move (S) over the instrument insert, wherein the at least two working ends are structurally biased to an open state, wherein the instrument insert is actuated by the outer tube being advanced distally over the instrument insert to cause the outer tube to urge the at least two working ends to a closed state. The surgical device (1) further comprises an optical fiber (60) for intraoperative sensing, wherein the optical fiber is arranged relative to the instrument insert to have axial forward visibility when the at least two working ends are in the closed state.

## Description

### TECHNICAL FIELD

The presently disclosed subject matter relates to a surgical device for use in microsurgery, and to a method of manufacturing the surgical device.

### BACKGROUND

Microsurgical instruments are typically designed for minimally invasive procedures and intended to execute precision tasks within confined areas. For example, in the case of intraocular surgery, these precision requirements are particularly critical in posterior ophthalmic surgeries, such as vitreoretinal procedures. To meet these requirements, microsurgical instruments such as intraocular instruments generally comprise a slender shaft or needle fitted with specialized working ends that enable grasping, manipulating, or severing tissues. For example, the working ends may comprise jaws or blades. The ability to grasp, manipulate, or sever structures within the eye's interior is essential across a range of ophthalmic procedures.

To facilitate these functions, microsurgical instruments employ relatively small outer diameters. For example, current intraocular surgical instruments are typically fabricated with outer diameters smaller than 0.6 mm, allowing for sutureless procedures in which incision wounds are sufficiently small to eliminate the need for postoperative suturing. Common instrument sizes include 23-gauge (G), 25G, and 27G, corresponding to outer diameters of 0.6 mm, 0.5 mm, and 0.4 mm, respectively.

However, the miniature size of these instruments introduces engineering challenges, particularly in achieving mechanical actions at the working ends, such as the closing of forceps jaws. Conventional hinged mechanisms that could support such actions are impractical at these compact scales. Consequently, a design has emerged whereby the working ends, such as the jaws of forceps, are biased to remain naturally open. When it is desired to close the working ends, an outer tube is advanced over the working ends, causing the jaws to converge and close. An exemplary configuration includes an inner rod holding the working ends at the distal tip, with the proximal end of the inner rod being secured to the main instrument body, ensuring stability of the working ends relative to the instrument. Given that the main body typically serves as the handle held by the surgeon, this design provides predictable operation since the working ends are kept at a fixed position relative to the handle. The closing action may be activated by a user-controlled mechanism on the handle, which allows controlled advancement of the outer tube, enabling the surgeon to precisely manipulate the working ends, for example to grasp a structure by moving the ends together.

There is a need to further improve upon microsurgical instruments of the type described above.

### SUMMARY

In a first aspect of the presently disclosed subject matter, a surgical device is provided for use in microsurgery, comprising:
- an outer tube comprising a distal end;
- an instrument insert extending axially from the distal end of the outer tube, wherein the instrument insert comprises at least two working ends, wherein the outer tube is configured to slidably move over the instrument insert, wherein the at least two working ends are structurally biased to an open state, wherein the instrument insert is actuated by the outer tube being advanced distally over the instrument insert to cause the outer tube to urge the at least two working ends to a closed state; and
- an optical fiber for intraoperative sensing, wherein the optical fiber is affixed and arranged relative to the instrument insert to have axial forward visibility when the at least two working ends are in the closed state.

The surgical device provides an instrument insert with at least two working ends. The instrument insert may normally extend from a distal end of an outer tube. In some embodiments, the instrument insert may be connected to, or formed by, a distal end of an inner rod that runs through the outer tube. The working ends may be adapted to serve various surgical functions, such as grasping, cutting, or holding tissue. In some embodiments, the working ends may take the form of jaws, blades, or other structures suited to the intended microsurgical procedure. In some examples, the working ends may be identical in form, such as a pair of identical jaws, to provide symmetrical functionality. In other embodiments, the working ends may differ in form, allowing them to complement each other in performing a specific surgical function.

The working ends may be structurally biased to remain in an open state when not acted upon by an actuation mechanism. This natural bias towards an open state may mean that, under normal conditions, the working ends do not engage each other. To actuate the working ends, the outer tube may be configured to slide over the instrument insert, thereby urging the working ends into a closed state. In some embodiments, the outer tube may apply a mechanical force onto parts of the working ends that protrude beyond the axial projection of the outer tube, thereby pressing the working ends towards each other. The outer tube may thus function as part of the actuation mechanism to actuate the working ends. It is noted that the instrument insert may be designed such that full or sufficient actuation of the working ends may be achieved by the outer tube advancing only a limited distance over the working ends. This limited advancement of the outer tube may apply sufficient force to close the working ends while minimizing the extent to which they are covered by the outer tube. By restricting the outer tube's movement, the working ends may remain largely unobstructed, thereby reducing potential interference with tissue manipulation or other surgical actions.

It may be desirable to have sensing capabilities during microsurgery, for example to provide real-time feedback on the surrounding structures. However, sensors directly attached to the distal end of the instrument may be bulky, which may limit maneuverability and obstruct the surgeon's view in the confined microsurgical space. To address this, the surgical instrument is provided with an optical fiber, which allows for remote sensing while maintaining a relatively slim instrument profile. Namely, the optical fiber may be configured to transmit signals between a sensor located outside the patient and the surgical site, enabling intraoperative sensing without adding bulk to the instrument's distal end. However, it has been recognized that the optical fiber may be limited or entirely blocked from forward visibility by the working ends, particularly when the working ends are in a closed state. Accordingly, to provide continued sensing capabilities even when the working ends are in a closed state, the optical fiber and the instrument insert are configured to maintain axial forward visibility when the working ends are closed. This configuration allows the optical fiber to capture and/or transmit signals directly forward along the instrument's longitudinal axis, ensuring effective sensing whether the working ends are open or closed.

The above-described configuration may enhance procedural accuracy by allowing the surgeon to receive real-time data on the area directly in front of the instrument during critical actions, such as grasping or manipulating tissues. In some embodiments, if used with a distance or OCT sensor, the configuration may allow continuous monitoring of distance or tissue structure, enabling the surgeon to make informed adjustments even when the working ends are mutually engaged.

Optionally, the instrument insert comprises a junction from which the at least two working ends originate, wherein the optical fiber extends longitudinally through the instrument insert and projects outwardly from the junction. The two working ends may originate from a junction, which may be formed as a centralized structural point within the insert. The optical fiber may extend longitudinally through the instrument insert and project outwardly from this junction. To facilitate this projection, the optical fiber may run within the outer tube up to the instrument insert. This arrangement may protect the optical fiber and help prevent unnecessary bulk, which could otherwise occur if the optical fiber were positioned alongside the outer tube.

Optionally, the optical fiber is concentrically aligned with the instrument insert at the junction. Thereby, the optical fiber may be centrally arranged within the instrument. This concentric alignment may enhance forward visibility by keeping the fiber aligned along the instrument's central axis, thereby optimizing its line of sight.

Optionally, the at least two working ends comprise an opening that is axially aligned with the distal end of the optical fiber when the at least two working ends are in the closed state. The working ends, when closed, may hinder forward visibility if no countermeasures are taken, in particular when the optical fiber projects from the instrument insert behind the working ends. To mitigate this, an opening may be included within the closed working ends, allowing forward visibility by the opening being longitudinally aligned with the optical fiber. The function of the working ends may remain unimpaired or only minimally affected, as the opening may have a limited size.

Optionally, the at least two working ends are configured to diverge laterally from a longitudinal axis of the instrument insert as they extend distally from the junction, thereby creating an unobstructed axial path for the optical fiber. As an alternative to including an opening within the closed working ends, the working ends may be configured to extend outwardly at an angle from the longitudinal axis of the instrument insert as they project distally from the junction. This arrangement results in an eccentric or asymmetrical positioning of the optical fiber and working ends in the transverse cross-section of the surgical instrument, where they may be laterally aligned when viewed from the distal end of the surgical instrument. This configuration may prevent the working ends from impairing forward visibility and may allow the use of existing working end designs without modifications, such as adding an opening.

Optionally, the outer tube comprises an inner tube, wherein the instrument insert is formed from a distal end of the inner tube to form an integral structure with the inner tube. In this configuration, the instrument insert may not be a separate part but rather manufactured directly from the inner tube. This may result in the instrument insert and the inner tube being a single, unified structure, which may offer advantages for manufacturability and enhance the mechanical properties of the surgical device.

Optionally, the inner tube and the instrument insert are manufactured from a single thick-walled tube, wherein the inner tube is formed by circumferentially reducing a diameter of a longitudinal portion of the thick-walled tube, wherein the instrument insert is formed in its open state from the thick-walled tube. This way, a single, unified structure is provided, with the working ends naturally biased to the open state, avoiding the need for additional components or manufacturing steps to achieve this bias.

Optionally, the inner tube and/or the instrument insert are formed from the single thick-walled tube using electrical discharge machining (EDM). EDM, in general, is well suited for creating the geometries required in the inner tube and instrument insert. Various EDM techniques may be used, including but not limited to wire EDM, die-sinking EDM, and hole-drilling EDM. Additionally, other electroforming methods may be used, depending on the required geometries and material properties.

Optionally, the instrument insert is formed in its closed state from the distal end of the inner tube and then biased into the open state.

Optionally, the outer tube comprises an inner tube or inner rod, wherein the instrument insert is axially welded to a distal end of the inner tube or inner rod.

Optionally, the at least two working ends are arranged symmetrically with respect to a longitudinal axis of the instrument insert.

Optionally, the at least two working ends comprise:
- a first working end functioning as a substantially fixed working end, wherein the first working end extends laterally from a longitudinal axis of the outer tube and maintains its position and orientation in the open state and the closed state; and
- a second working end functioning as a movable working end, wherein the second working end diverges laterally from the longitudinal axis of the outer tube in the open state, wherein the second working end is urged towards the first working end when the outer tube is advanced distally over the instrument insert.

In this configuration, one working end remains stationary, while the other working end moves to engage with the former. The stationary working end may also be referred to as a fixed working end while the other working end may be referred to as a moving working end. By having one working end remain stationary, a reliable reference point may be provided, which may reduce the risk of unintended tissue damage.

Optionally, the first working end comprises a lumen, wherein the optical fiber extends longitudinally through the lumen. As the first working end remains stationary, the first working end may support a lumen that houses the optical fiber while avoiding potential (bending) movement that could occur if the fiber were routed through the movable working end. This configuration may allow the optical fiber to extend to the distal tip or near the tip of the fixed working end, positioning the distal end of the optical fiber close to the surgical site for improved visibility while keeping most of the surgical device outside the fiber's field of view. Additionally, by running through the lumen, the optical fiber may remain well-protected, reducing the risk of damage during use.

Optionally, the surgical device further comprises an inner tube arranged in the outer tube, wherein one of the first working end and the second working end is formed by the inner tube and another one of the first working end and the second working end is welded to the inner tube or formed by an intermediate tube arranged between the inner tube and the outer tube. In some embodiments, the stationary working end may be formed by an inner tube arranged within the outer tube, for example, using EDM. The moving working end may be welded to the inner tube or manufactured from an intermediate tube positioned between the inner tube and the outer tube. Both techniques are cost-effective manufacturing methods. In an alternative embodiment, the moving working end may be formed by an inner tube arranged in the outer tube, and the stationary working end may be welded to the inner tube or formed by an intermediate tube arranged between the inner tube and the outer tube.

Optionally, the outer tube comprises an inner rod, wherein the instrument insert is axially welded to a distal end of the inner rod, wherein the optical fiber is arranged off-axially with respect to the instrument insert in a groove or lumen of the inner rod and projects outwardly from the distal end of the inner rod. This arrangement may allow for the use of existing instrument inserts, such as forceps jaws which may be axially welded to a wire forming an inner rod that runs through the outer tube. The optical fiber may be incorporated by running the optical fiber in a groove or lumen within the inner rod, allowing the optical fiber to extend outward in parallel with the working ends. This configuration may maintain forward visibility by positioning the fiber alongside the working ends without interfering with the working ends' operation.

Optionally, the instrument insert is eccentrically welded to the distal end of the inner rod, wherein the inner rod is arranged eccentrically within the outer tube so that the at least two working ends are centrically positioned with respect to a longitudinal axis of the surgical device. It may be desirable to position the working ends centrally within the outer tube, as this may align with the surgeon's intuitive expectations. Such central arrangement of the working ends may be achieved by welding the instrument insert centrally to the inner rod and arranging the inner rod centrally within the outer tube. Alternatively, however, the instrument insert may be welded off-center, or eccentrically, to the inner rod. This design may provide more space to accommodate the optical fiber and better preserve its field of view. By then arranging the inner rod eccentrically within the outer tube, the working ends can still be positioned centrally with respect to the longitudinal axis of the surgical device.

In a further aspect of the presently disclosed subject matter, a method is provided of manufacturing a surgical device, comprising:
- providing an outer tube comprising a distal end;
- providing an instrument insert which comprises at least two working ends, wherein the at least two working ends are structurally biased to an open state,
- mutually arranging the outer tube and the instrument insert so that the instrument insert extends axially from the distal end of the outer tube and the outer tube is slidably movable over the instrument insert to enable actuation of the instrument insert when the outer tube is advanced distally over the instrument insert to cause the outer tube to urge the at least two working ends to a closed state;
- providing an optical fiber for intraoperative sensing; and
- arranging the optical fiber relative to the instrument insert to have axial forward visibility when the at least two working ends are in the closed state.

Optionally, the method further comprises:
- providing an inner tube;
- arranging the inner tube in the outer tube; and
- forming the at least two working ends from the distal end of the inner tube using electrical discharge machining (EDM).

Optionally, the method further comprises:
- providing an inner tube;
- arranging the inner tube in the outer tube;
- forming the first working end from the inner tube;
- welding the second welding end to the inner tube.

Optionally, the method further comprises:
- providing an inner tube;
- arranging the inner tube in the outer tube;
- forming the first working end from the inner tube;
- providing an intermediate tube, arranging the intermediate tube between the inner tube and the outer tube, and forming the second working end from the intermediate tube.

It will be appreciated by those skilled in the art that two or more of the above-mentioned embodiments, implementations, and/or aspects of the presently disclosed subject matter may be combined in any way deemed useful.

All described manufacturability aspects of the surgical device may denote corresponding manufacturing steps within a method of manufacturing.

Modifications and variations of the surgical device or the manufacturing method, which align with the described modifications, variations, and optional aspects of each respective entity and activity, may be implemented by a person skilled in the art based on the present description.

### BRIEF DESCRIPTION OF THE DRAWINGS

These and other aspects of the presently disclosed subject matter are apparent from and will be elucidated with reference to the embodiments described hereinafter. In the drawings,
Figs. 1A and 1B show a distal part of a surgical device for intraocular surgery, comprising an instrument insert in the form of a forceps with two jaws, an outer tube, and an optical fiber, wherein the jaws, when closed, include an opening that is axially aligned with a distal end of the optical fiber to maintain axial forward visibility;
Fig. 1C shows an actuation of the instrument insert;
Figs. 2A-2C show an instrument insert where the jaws diverge laterally from a longitudinal axis of the instrument insert to create an unobstructed axial path for the optical fiber;
Figs. 3A and 3B show an instrument insert which may be manufactured from a thick-walled tube by wire electrical discharge machining (wire-EDM);
Figs. 4A and 4B show an instrument insert having a fixed jaw and a movable jaw, wherein the fixed jaw comprises the optical fiber;
Figs. 5A and 5B show an instrument insert in which the fixed jaw is manufactured from an inner tube and in which the movable jaw is obtained from a commercially available forceps and welded to the inner tube;
Figs. 6A and 6B show an instrument insert which comprises an inner tube which comprises, or forms, the fixed jaw and an intermediate tube which comprises or forms the movable jaw;
Figs. 7A and 7B show an instrument insert eccentrically welded to an inner tube, with the inner tube being eccentrically arranged within the outer tube to position the jaws centrally and the optical fiber extending off-axially from the inner tube;
Figs. 8A and 8B show an instrument insert in which the instrument insert is welded to an inner rod and the optical fiber runs through a groove of the inner rod;
Figs. 9A and 9B show an instrument insert derived from a commercially available forceps and arranged side-by-side with the optical fiber in the outer tube;
Figs. 10A-10C show an instrument insert which is inserted into a lumen of an inner rod and in which the optical fiber runs through another lumen of the inner rod;
Figs. 11A-1 1C show an instrument insert manufactured from an inner tube, wherein the jaws diverge symmetrically from a longitudinal axis of the instrument insert;
Figs. 12A-11C show an instrument insert manufactured from an inner tube, wherein the jaws comprise a fixed jaw and a movable jaw; and
Figs. 13A-11C show an instrument insert manufactured from an inner tube, wherein the jaws comprise a movable jaw and a fixed jaw comprising an opening aligned with a distal end of the optical fiber to maintain axial forward visibility.

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- 1-10, 100-102: surgical device
- 20-29, 120-122: outer tube
- 30-39, 130-132: instrument insert
- 40-49, 140-142: first working end
- 50-59, 150-152: second working end
- 60-69, 160-162: optical fiber
- 70,71, 170: opening in working end(s)
- 72: contact surface
- 73: junction
- 74: opening
- 75-77, 175-177: inner tube
- 78, 79: lumen
- 80: intermediate tube
- 81: inner tube
- 82: eccentric lumen for optical fiber
- 83: inner rod
- 84: groove
- 85, 86: multi-lumen guide
- 87: groove
- 88-90: lumen
- L: longitudinal axis
- S: slideable movement

### DESCRIPTION OF EMBODIMENTS

The following describes the presently disclosed subject matter with reference to the microsurgical device as an intraocular surgical device. However, this is not limiting, as the surgical device may also be used in other types of microsurgery, such as neurosurgery, otologic surgery, vascular microsurgery, or other delicate tissue procedures requiring high precision and minimally invasive techniques. Any reference in this specification to 'intraocular' or the eye is to be understood as equally applicable to other types of microsurgery, unless otherwise precluded by the context.

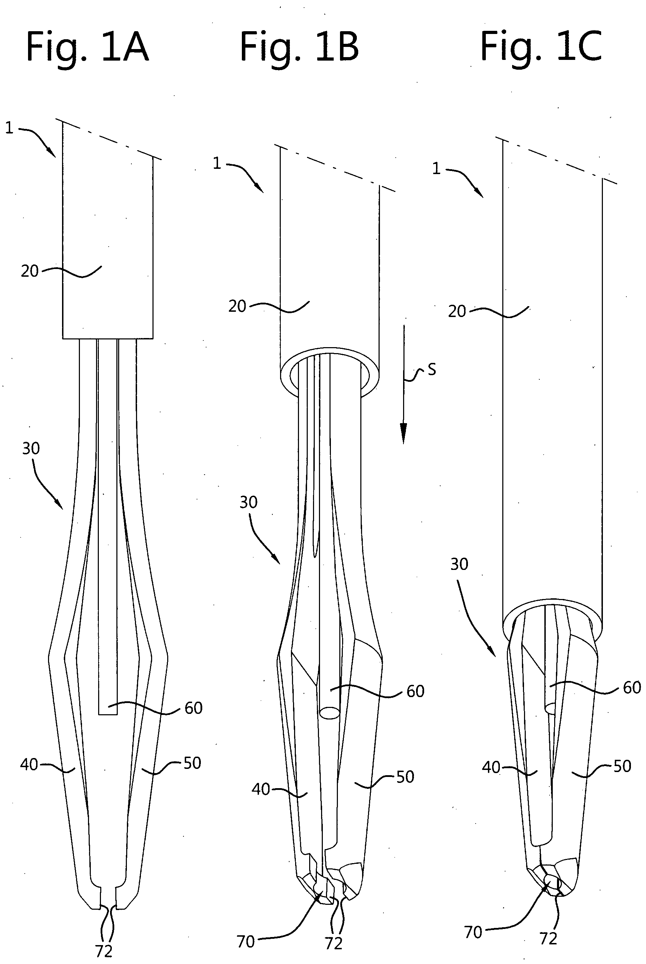

The following describes, with reference to the figures, various embodiments of surgical devices for use in intraocular surgery. The surgical devices may generally include an outer tube with a distal end configured for insertion into an eye during a surgical procedure and an instrument insert extending axially from the distal end of the outer tube. The instrument insert comprises at least two working ends which may be structurally biased to an open state. As further described with reference to Fig. 1C, actuation of the working ends may be achieved by slidably moving the outer tube over the instrument insert. In some examples, the instrument insert may remain stationary along its longitudinal axis during this movement, for example by being affixed to an inner tube or inner rod, which may, at its proximal end, be connected to a handle body of the surgical device. In such examples, the outer tube may be configured to move relative to the handle body, and when advanced distally over the instrument insert, the outer tube may urge the at least two working ends into a closed state.

In the illustrated examples, the instrument insert is shown as a forceps with two jaws. However, this is merely exemplary, as the working ends may take various other forms, such as the blades of scissors, pincers, or graspers, depending on the intended surgical application. The surgical device may itself be considered a surgical instrument. The term 'instrument insert' in this context may refer to the instrument's component extending from the distal end of the outer tube including the working ends.

The described surgical devices may further incorporate an optical fiber for intraocular sensing. The optical fiber is arranged relative to the instrument insert to provide axial forward visibility even when the at least two working ends are in the closed state. The optical fiber may be optimized for specific intraocular sensing applications. In some embodiments, when used with an OCT sensor, the optical fiber may be a single-mode fiber and may include a lens at its distal end. This configuration may allow OCT signals to be transmitted through the optical fiber, emerge from its distal end in a focused manner, interact the target tissue, and be reflected back through the fiber to the OCT sensor for analysis. However, the optical fiber is not limited to OCT but may also be used for other intraocular sensing modalities, either with or without a lens.

**Figs. 1A** to **1C** show a distal part of a surgical device 1 designed for use in intraocular surgery. The surgical device 1 may include an outer tube 20, an instrument insert 30, and an optical fiber 60. The instrument insert 30 may be axially positioned within, and extend distally from, the outer tube 20 and includes at least two working ends, shown as jaws 40 and 50. These jaws may be structurally biased to an open state, but may be actuated to a closed state when the outer tube 20 is advanced distally, sliding over the instrument insert 30 and exerting an inward radial force. The sliding movement S is schematically illustrated in Fig. 1B by an arrow, while the resulting closed state of the jaws 40, 50 is depicted in Fig. 1C. In this respect, it is noted that the fully open state of the jaws may have a wider divergence between the jaws than that shown in Figs. 1A and 1B, for example to facilitate grasping actions. The extent of this divergence may depend on the structural bias of the jaws.

The jaws 40 and 50 may include contact surfaces 72, through which the jaws may mutually engage when in the closed state, e.g., to grasp tissue. The contact surfaces 72 may be configured to define an opening 70 when the jaws are closed. This opening 70 may be formed as a circular or elliptical aperture by two half-circle sections which are formed between the contact surfaces 72 of each jaw. The opening 70 may also be axially aligned with the distal end of the optical fiber 60, allowing for forward axial visibility for intraocular sensing. The optical fiber 60 may be longitudinally arranged relative to the instrument insert 30, extending through the length of the instrument insert and projecting outwardly from a junction (not shown in Figs. 1A and 1B) where the working ends originate. This configuration may provide axial visibility through the opening 70 in the closed state, as depicted in Fig. 1C.

The instrument insert 30 of the surgical device 1 may be manufactured from a thick-walled tube having a channel to accommodate the fiber 60. The tube may be reduced to size by grinding or turning along its length and further processed using methods such as wire-EDM to form the jaws. The resulting design may be robust with material thicknesses exceeding 60 µm. The dimensional considerations may allow the surgical device to retain a compact profile. In some embodiments, the surgical device may have a 23G profile, with an inner forceps diameter of approximately 200 µm and an outer tube diameter of 600 µm, while still accommodating the optical fiber and its shielding.

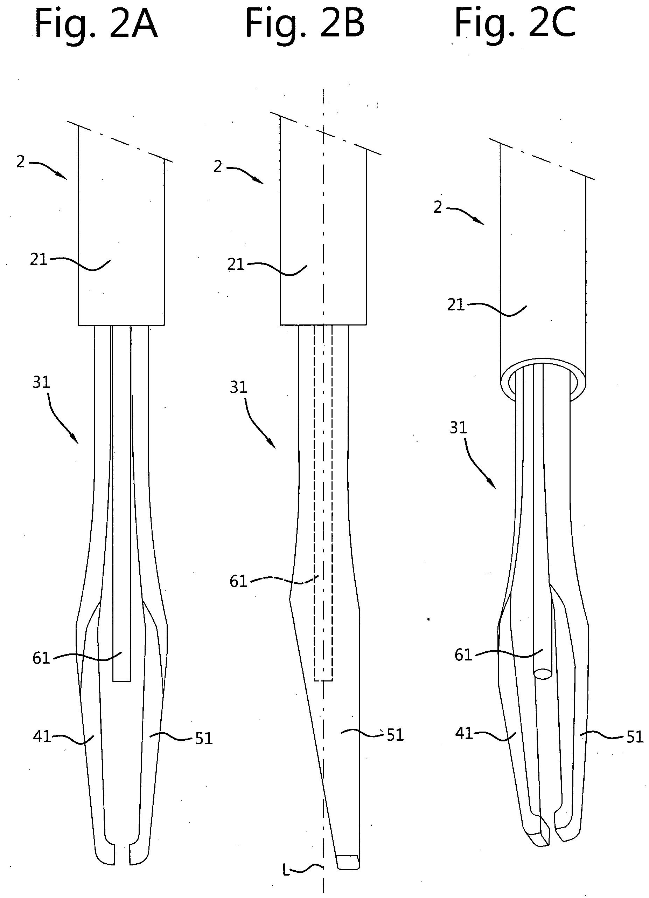

The embodiments depicted with reference to Figs. 2A-13C show surgical devices without additionally illustrating the actuation to a closed state. However, as in the embodiment of Figs. 1A-1C, actuation may be achieved by advancing the outer tube distally, causing it to slide over the instrument insert and exert an inward radial force. The sliding movement S, schematically illustrated in Fig. 1B by an arrow, is therefore not repeated in these figures but applies similarly to these embodiments.

**Figs. 2A** to **2C** show the distal part of a surgical device 2. As in the previous embodiment of Figs. 1A to 1C, the surgical device 2 includes an outer tube 21, an instrument insert 31, and an optical fiber 61. The instrument insert 31 may be axially positioned within and extends from the outer tube 21 and may include jaws 41 and 51. The optical fiber 61 may be positioned centrally within the instrument insert 31. In this example, the jaws 41 and 51 are configured to diverge laterally from the longitudinal axis L of the instrument insert 31, which is substantially the axis of the optical fiber 61, as the jaws extend distally from the outer tube 21. This configuration creates an unobstructed axial path for the optical fiber 61, as shown in Figs. 2A to 2C, and allows the optical fiber 61 to maintain unobstructed forward visibility along longitudinal axis L. The design of the instrument insert 31 and jaws 41 and 51 may thus result in an asymmetric configuration of the jaws and the optical fiber 61, which contrasts with the concentric and symmetric design depicted in Figs. 1A to 1C. Due to this axially asymmetric configuration, the contact surfaces of the jaws do not require an opening when the jaws are closed to maintain axial visibility in the closed state of the instrument insert 31. Instead, the contact surfaces of the jaws may fully engage, forming a continuous closure. As a result, the contact surfaces of the jaws may correspond to those of commercially available jaws, such as off-the-shelf forceps jaws.

The jaws 41 and 51 diverge laterally to accommodate the optical fiber 61 within the same axial plane. The surgical device 2 may be manufactured similarly to the symmetric design shown in Figs. 1A to 1C, with modifications to accommodate the asymmetric design. The thick-walled tube forming the instrument insert 31 may be processed by grinding or turning, followed by two-degree-of-freedom wire-EDM to form the tip with the desired lateral divergence.

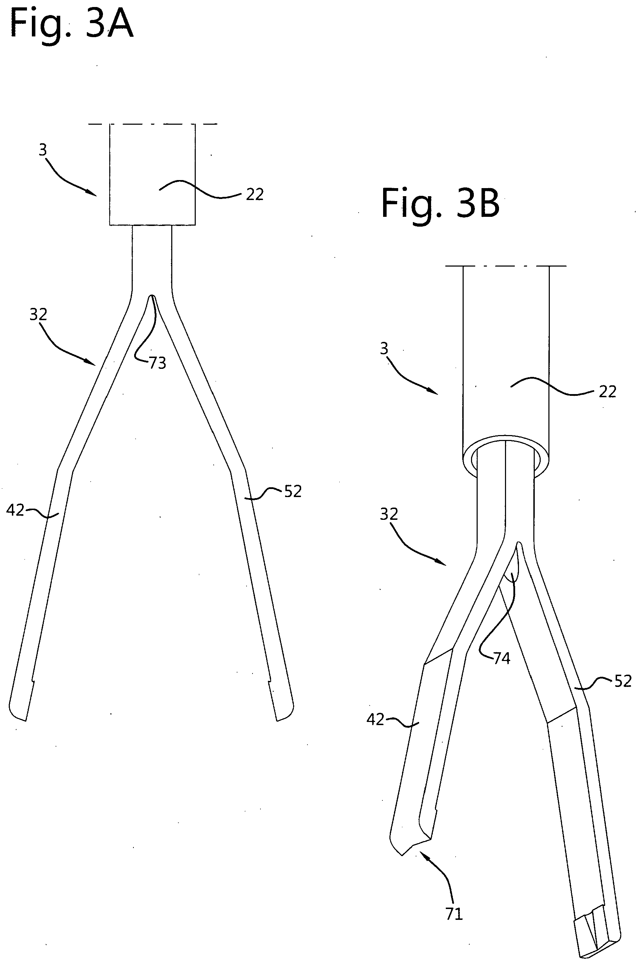

**Figs. 3A** and **3B** show the distal part of a surgical device 3, comprising an outer tube 22, an instrument insert 32, and jaws 42 and 52. The instrument insert 32 comprises a junction 73 from which the jaws 42 and 52 originate. An opening 71 is formed between the jaws 42 and 52 in their closed state, which opening 71 may be axially aligned with the distal end of the optical fiber extending through the instrument insert 32. The junction 73, explicitly shown in this example, provides a central location where the optical fiber may project outwardly through an opening 74. The instrument insert 32 may be manufactured from a thick-walled tube using wire-EDM. In this example, wire-EDM may be applied along the entire length of the tube to create an inner tube that fits within the outer tube and from which the manufactured jaws 42, 52 protrude. The jaws 42 and 52 may initially be formed in an almost closed state and subsequently split and biased into an open state. Any sharp edges resulting from the wire-EDM process may be finished to achieve the required surface quality.

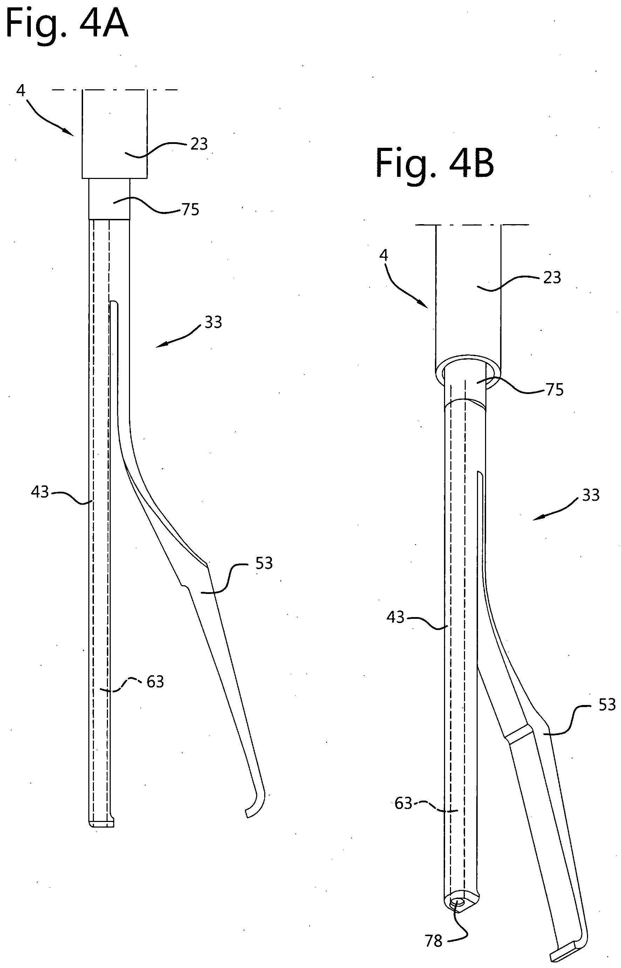

**Figs. 4A** and **4B** show the distal part of a surgical device 4 comprising an outer tube 23, an instrument insert 35, a fixed jaw 43, a movable jaw 53, and an optical fiber 63. The fixed jaw 43 may extend laterally from the longitudinal axis of the outer tube 23 and remain stationary during operation, functioning as a fixed working end. The movable jaw 53 may diverge laterally in the open state, as depicted in Figs. 4A and 4B, and may be urged towards the fixed jaw 43 in the closed state when the outer tube 23 is advanced distally over the instrument insert 35. The fixed jaw 43 may include a lumen 78 through which the optical fiber 63 extends longitudinally. The optical fiber 63 may protrude at the distal end of the fixed jaw 43 through an opening in the lumen 78, ensuring robust alignment and forward visibility. This configuration may allow the optical fiber to be positioned close to the tip of the fixed jaw 43, protected within the lumen 78 and substantially unaffected by the movement of the movable jaw 53.

The instrument insert 35 may be manufactured as a monolithic piece using microforming techniques like die-sink EDM or wire-EDM for cutting and EDM drilling for the lumen 78. The instrument insert 35, including the fixed and movable jaws, may be axially welded to an inner tube 75 arranged within the outer tube 23 to ensure structural integrity. The grasping mechanism involves only the movable jaw 53, which closes against the fixed jaw 43. This approach may provide advantages over designs with double-closing jaws, as it reduces complexity while retaining proven grasping performance typical of state-of-the-art designs. The fixed jaw 43's lumen may ensure that the optical fiber remains secure and stationary, for example to provide reliable and precise distance measurement.

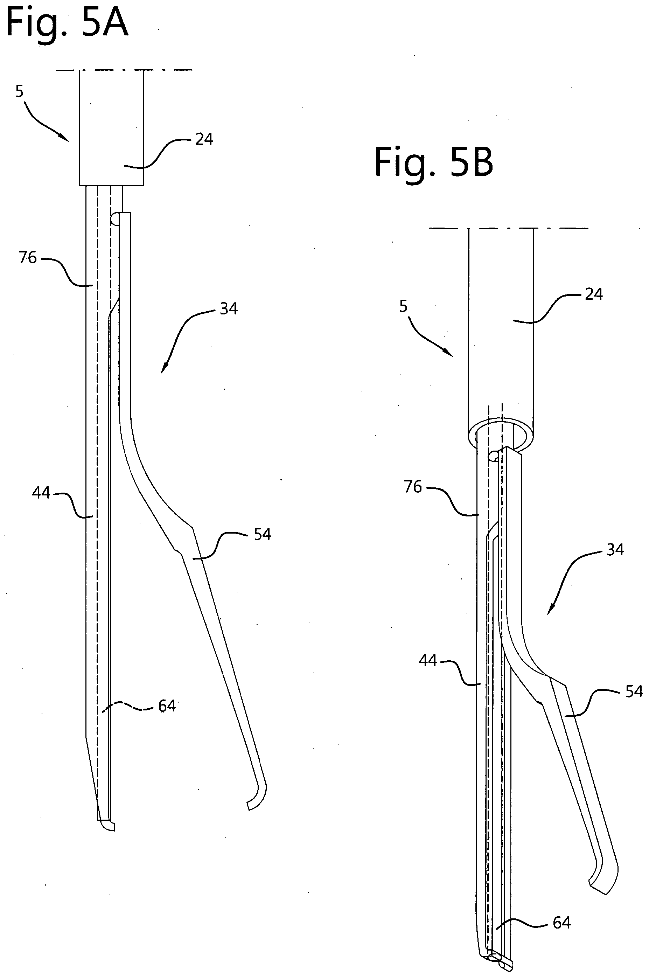

**Figs. 5A** and **5B** show the distal part of a surgical device 5, comprising an outer tube 24, an instrument insert 34, a fixed jaw 44, a movable jaw 54, and an optical fiber 64. The fixed jaw 44 is formed from the distal end of an inner tube 76 arranged within the outer tube 24 and extends outward from the outer tube 24 along its longitudinal axis. The fixed jaw 44 maintains its position and orientation during operation, functioning as a stationary working end. The movable jaw 54 may diverge laterally in the open state, as shown in Figs. 5A and 5B, and may be urged towards the fixed jaw 44 in the closed state when the outer tube 24 is advanced distally over the instrument insert 34. The fixed jaw 44 includes a lumen through which the optical fiber 64 extends longitudinally, emerging from a distal opening in the fixed jaw 44. This way, the optical fiber 64 may remain stationary and protected within the lumen.

In some examples, the movable jaw 54 may be separately manufactured and welded or bonded to the inner tube 76. This configuration allows for the use of a commercially available, e.g., off-the-shelf, forceps jaw, which may be modified to include only the movable component. Compared to the axial welded version shown in Figs. 4A and 4B, the fixed jaw 44 is formed from the inner tube 76 itself and may, for example, be shaped by wire-EDM cutting. As in the example of Figs. 4A and 4B, the grasping mechanism involves the movable jaw 54 closing against the stationary fixed jaw 44. This single-jaw closing configuration may reduce complexity compared to double-closing jaw designs while still achieving effective grasping performance.

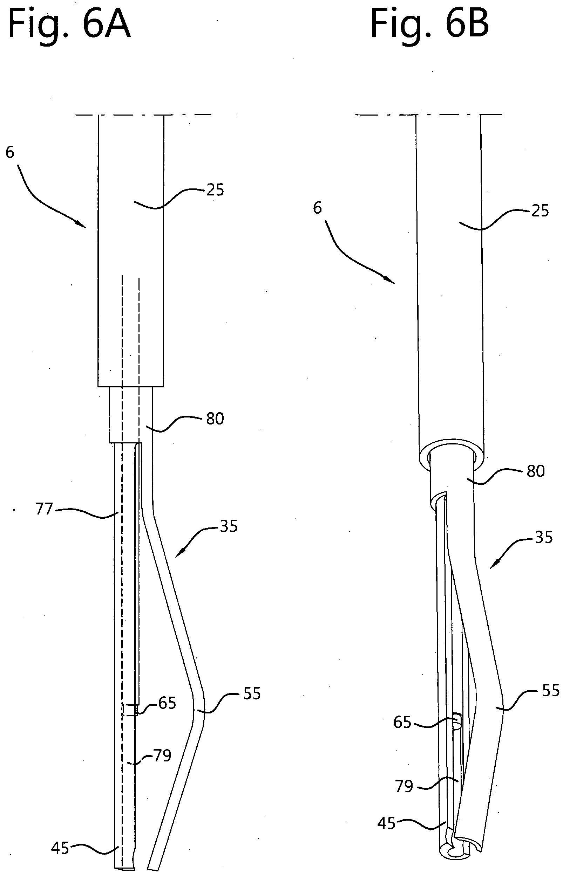

**Figs. 6A** and **6B** show the distal part of a surgical device 6, comprising an outer tube 25, an instrument insert 35, a fixed jaw 45, a movable jaw 55, and an optical fiber 65. The fixed jaw 45 is formed from the distal end of an inner tube 77, which is arranged within the outer tube 25. The movable jaw 55 is formed from the distal end of an intermediate tube 80, which is arranged concentrically between the inner tube 77 and the outer tube 25. The intermediate tube 80 may be affixed to the inner tube 77 or otherwise constrained to prevent axial movement during actuation of the surgical device 6, ensuring that the jaws 45 and 55 maintain longitudinal alignment throughout operation. The fixed jaw 45 includes a lumen 79 through which the optical fiber 65 extends longitudinally. The optical fiber 65 emerges from a distal opening in the fixed jaw 45, ensuring robust alignment and protection during operation. The fixed jaw 45 may function as a stationary working end, maintaining its position and orientation in both the open and closed states. The movable jaw 55 diverges laterally in the open state, as depicted in Figs. 6A and 6B, and is urged towards the fixed jaw 45 in the closed state when the outer tube 25 is advanced distally over the instrument insert 35.

This example differs from the previous embodiment of Figs. 5A and 5B by utilizing an intermediate tube 80 to form the movable jaw 55. This three-tube configuration may leverage standard production techniques. In some embodiments, the intermediate tube 80 may be partially cut and shaped, for example using wire-EDM, to create a wedge-shaped movable jaw. The movable jaw 55 may close towards the fixed jaw 45 in a manner similar to the single fixed jaw configurations described previously.

In an alternative configuration to that shown in Figs. 6A and 6B, the jaws are formed by the inner tube and the intermediate tube but with their roles switched. In particular, the stationary jaw may be formed from the distal end of the intermediate tube, which may be arranged concentrically between the inner tube and the outer tube, while the movable jaw may be formed from the distal end of the inner tube. This arrangement may still maintain the three-tube configuration while reversing the roles of the inner and intermediate tubes to achieve an actuation mechanism. In this alternative configuration, the stationary jaw formed from the intermediate tube may provide a fixed reference point, and the movable jaw formed from the inner tube may be urged towards the stationary jaw when the outer tube is advanced distally over the instrument insert.

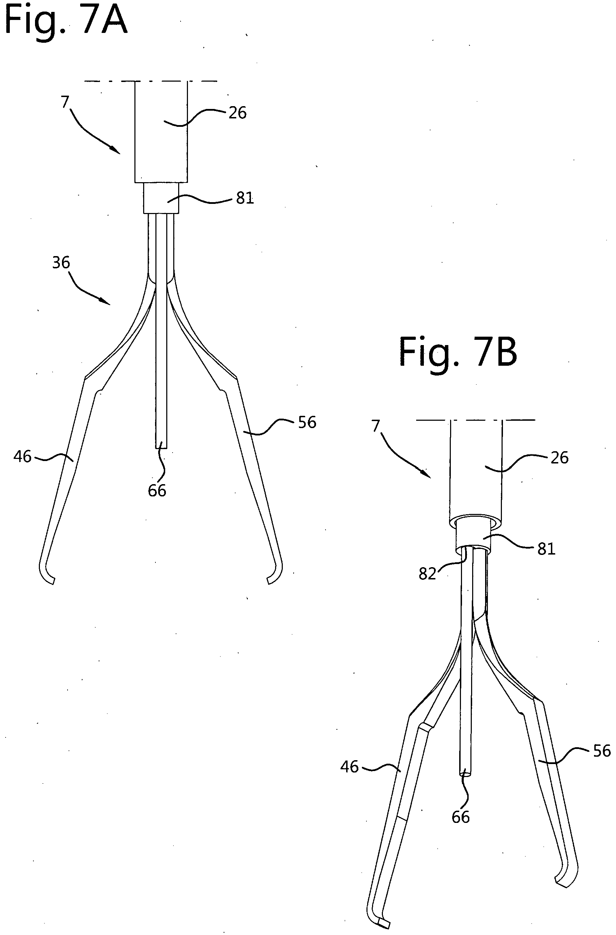

**Figs. 7A** and **7B** show the distal part of a surgical device 7, comprising an outer tube 26, an instrument insert 36, jaws 46 and 56, an optical fiber 66, and an inner tube 81. The inner tube 81 is eccentrically arranged within the outer tube 26 and includes an eccentric lumen 82 that houses the optical fiber 66. The instrument insert 36, which includes the jaws 46 and 56, may be eccentrically welded to the distal end of the inner tube 81. This arrangement may position the jaws 46 and 56 centrally relative to the longitudinal axis of the surgical device 7 while allowing the optical fiber 66 to extend off-axially through the eccentric lumen 82. The optical fiber 66 may project outwardly from the distal end of the inner tube 81. The fixed arrangement of the inner tube 81 and its eccentric lumen 82 may ensure that the optical fiber 66 is securely housed and protected while maintaining precise alignment for intraocular sensing. The jaws 46 and 56 may remain centrally positioned with respect to the longitudinal axis of the surgical device, ensuring predictable behavior during operation. In the open state, the jaws may diverge laterally, as shown in Figs. 7A and 7B, and may be urged towards each other in the closed state when the outer tube 26 is advanced distally over the instrument insert 36. The design of the surgical device 7 may enable the use of standard forceps tip jaws, which may be welded to the inner tube 81, for example using a proven welding method. The eccentric lumen 82 facilitates holding the optical fiber 66 in an off-axis position while ensuring that the jaws 46 and 56 remain centrally aligned. This configuration may prevent parasitic motion during closing, such as lateral shifting due to uneven resistance, and may ensure consistent and effective grasping performance. Additionally, this design may avoid the need for side-by-side tubing.

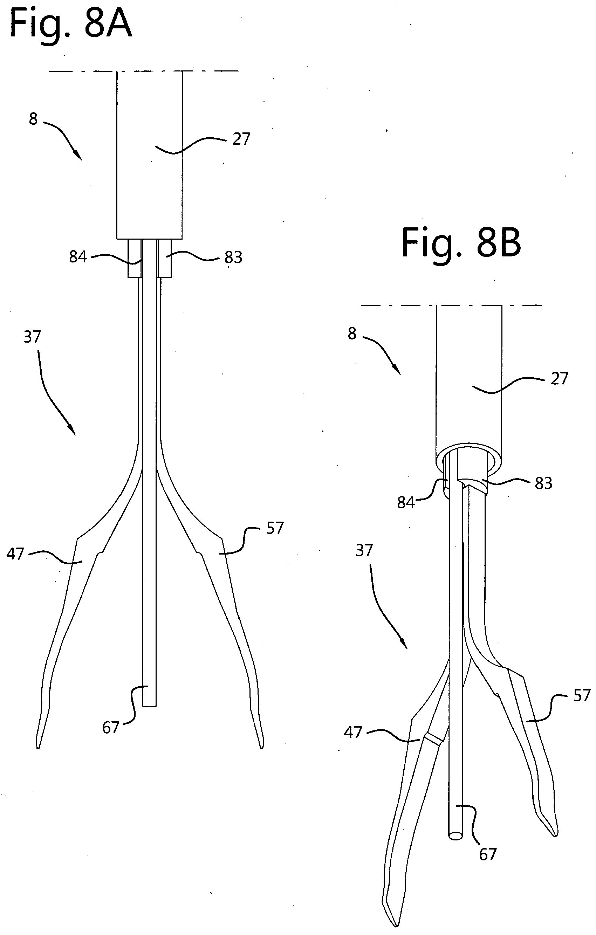

**Figs. 8A and 8B** show the distal part of a surgical device 8, comprising an outer tube 27, an instrument insert 37, jaws 47 and 57, an optical fiber 67, and an inner rod 83. The inner rod 83 may extend through the outer tube and includes a groove 84 that houses the optical fiber 67. The instrument insert 37, which includes the jaws 47 and 57, is welded to the distal end of the inner rod 83. The optical fiber 67 is arranged off-axially within the groove 84 of the inner rod 83 and may project outwardly from the distal end of the inner rod. The groove 84 may be formed along the surface of the inner rod 83, for example using laser grinding technology, to securely hold the optical fiber 67. To further protect and stabilize the optical fiber, a thin tube (not shown), for example made of plastic, may enclose the inner rod 83 and optical fiber 67. This thin tube may keep the optical fiber 67 in place and shielded during operation.

As in the example of Figs. 7A and 7B, the jaws 47 and 57 are eccentrically welded to the distal end of the inner rod 83. This configuration may position the jaws 47 and 57 centrally with respect to the longitudinal axis of the surgical device 8, while the optical fiber 67 remains positioned off-axis within the groove 84. This design is similar to that of Figs. 7A and 7B, but replaces the inner tube with an inner rod. The use of an inner rod offers similar production advantages, with the groove replacing the eccentric lumen for fiber housing. The jaws may be welded to the inner rod using a proven welding method, ensuring compatibility with existing manufacturing techniques.

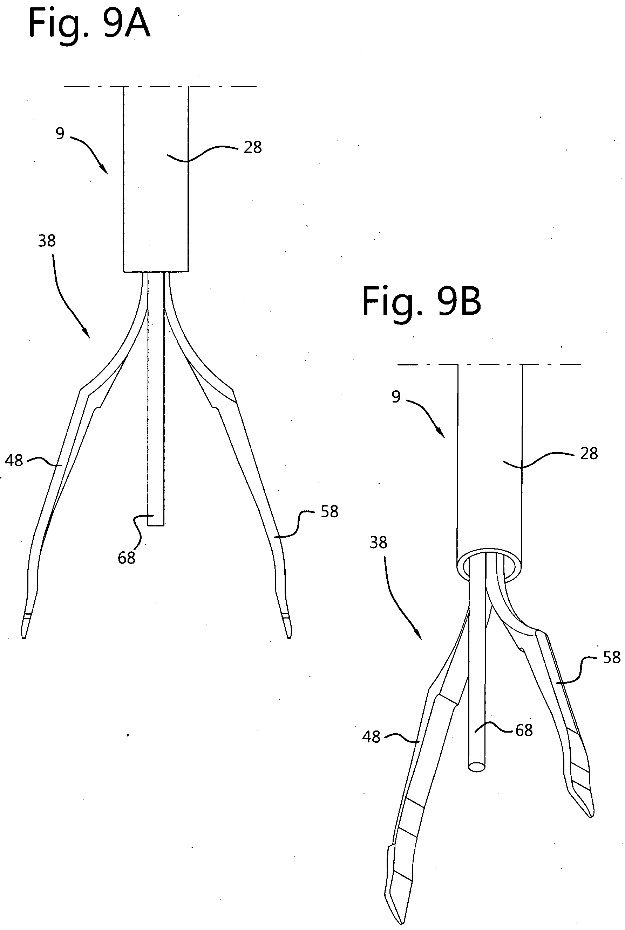

**Figs. 9A** and **9B** show the distal part of a surgical device 9, comprising an outer tube 28, an instrument insert 38, jaws 48 and 58, and an optical fiber 68. The instrument insert 38 may be derived from a commercially available forceps, with the jaws 48 and 58 mounted on an inner rod that extends through the outer tube 28. The optical fiber 68 may be arranged side-by-side with the instrument insert 38 within the outer tube 28 and may emerge alongside the inner rod from the distal end of the outer tube. In this configuration, the instrument insert 38 and optical fiber 68 are positioned parallel to each other. To facilitate the integration of the optical fiber 68, a groove may be formed along the surface of the inner rod to house the optical fiber 68. In some embodiments, the groove may be created as an additional processing step during modification of the commercially available forceps. The use of a commercially available forceps, modified with a groove in the inner rod, may offer manufacturing advantages by reducing the need for entirely custom parts. The optical fiber 68 may remain protected within the outer tube 28 while being positioned close to the surgical site for effective sensing.

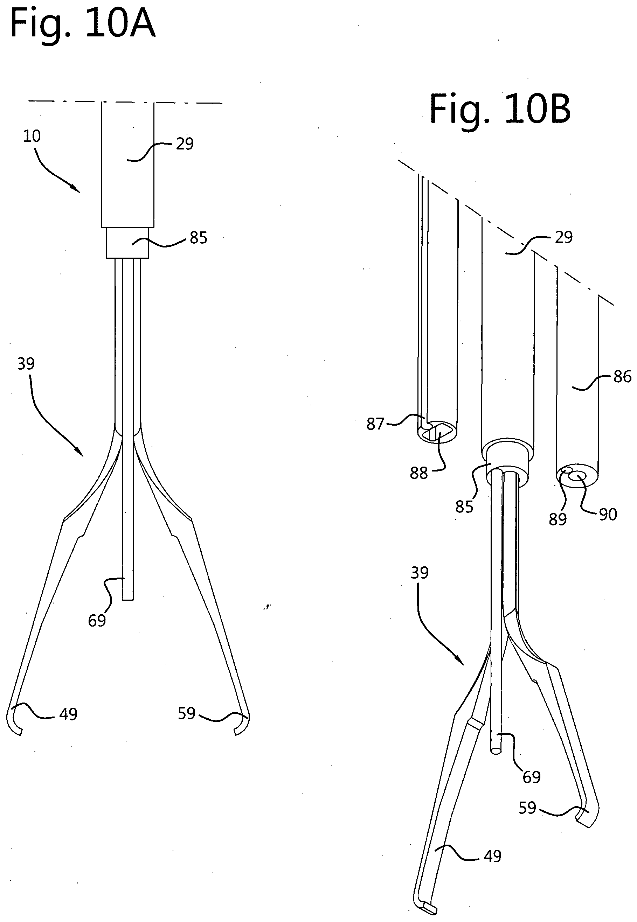

**Figs. 10A-10C** show the distal part of a surgical device 10, comprising an outer tube 29, an instrument insert 39, jaws 49 and 59, an optical fiber 69, and a multi-lumen guide, shown as either guide 85 or guide 86 in Fig. 10B. The multi-lumen guide, which serves as an inner rod and may extend through the outer tube 29, is configured to securely house both the instrument insert 39 and the optical fiber 69.

Specifically, the multi-lumen guide 85 may include a groove 87 to hold the optical fiber 69 and a lumen 88 to house the instrument insert 39. Alternatively, the multi-lumen guide 86 may include a lumen 89 for the optical fiber 69 and a separate lumen 90 for the instrument insert 39. In both configurations, the instrument insert 39 is positioned within the guide and welded to its distal end. The optical fiber 69 runs through the respective groove or lumen and projects outwardly from the distal end of the multi-lumen guide. The jaws 49 and 59 are mounted on the instrument insert 39, which is eccentrically positioned within the multi-lumen guide. This arrangement allows the jaws to remain centrally aligned with the longitudinal axis of the surgical device 10 while the optical fiber 69 is offset within its dedicated groove or lumen. In the open state, the jaws 49 and 59 may diverge laterally, as shown in Figs. 10A and 10B, and are urged towards each other to establish the closed state when the outer tube 29 is advanced distally over the instrument insert 39. The use of a multi-lumen guide provides a compact and efficient means of housing both the instrument insert 39 and the optical fiber 69 within a single structure. This approach enables the use of standard forceps tips while providing secure and convenient guidance for both the fiber and the forceps tip, thereby minimizing motion or misalignment during operation.

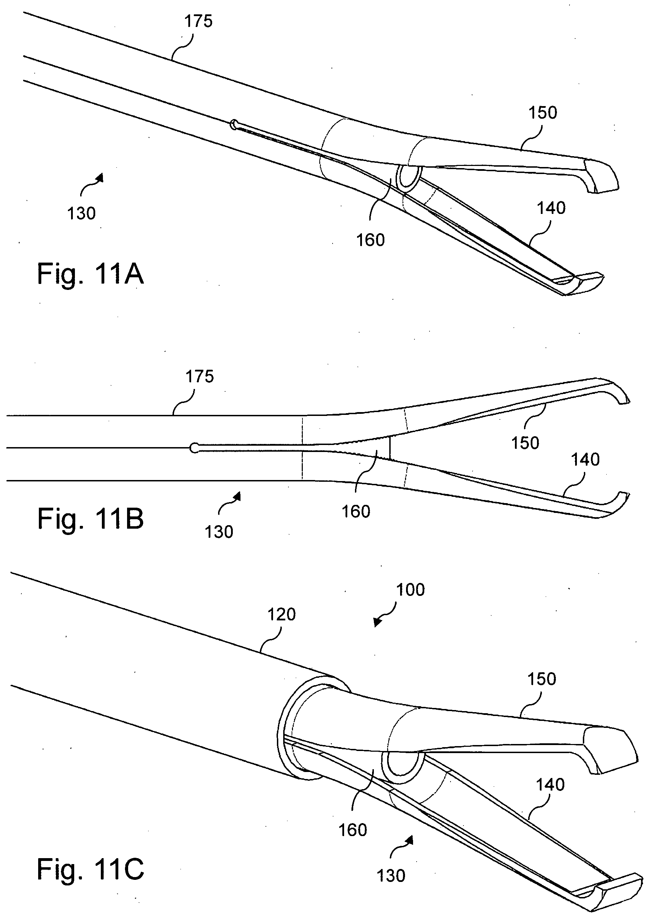

**Figs. 11A-11C** show a distal part of a surgical device 100 for use in intraocular surgery. The surgical device 100 includes an outer tube 120, an instrument insert 130, and an optical fiber 160. The instrument insert 130 may extend distally from the outer tube 120 and may include two working ends, shown as jaws 140 and 150. The instrument insert 130 may be formed from a single inner tube 175, with the jaws 140 and 150 manufactured directly from its distal end. The manufacturing process may for example involve wire electrical discharge machining (wire-EDM) to achieve the required structure. The jaws may be structurally biased to remain in an open state, as shown in Figs. 11A-11C, and may be actuated to a closed state by advancing the outer tube 120 distally over the instrument insert 130. The jaws 140 and 150 may be symmetrical, diverging laterally and symmetrically from the longitudinal axis of the instrument insert 130 when in the open state. The optical fiber 160 may be positioned within the inner tube 175 and may extend longitudinally through the instrument insert 130. The optical fiber 160 may project distally from the instrument insert to provide intraocular sensing capabilities. Although not directly shown in Figs. 11A-11C, the contact surfaces of the jaws 140 and 150, with which the jaws contact each other upon actuation, may be modified to maintain forward visibility from the optical fiber 160 even in the closed state of the jaws. In some embodiments, the contact surfaces may include a cut-out or opening to ensure that the optical fiber's line of sight remains unobstructed even when the jaws are in the closed state. Fig. 11A provides an isometric view of the instrument insert 130, showing the arrangement of the jaws 140 and 150 in the open state. Fig. 11B shows a side view, illustrating the symmetric divergence of the jaws. Fig. 11C shows an isometric view of the distal end of the surgical device 100, where the instrument insert 130 and the optical fiber 160 emerge from the outer tube 120.

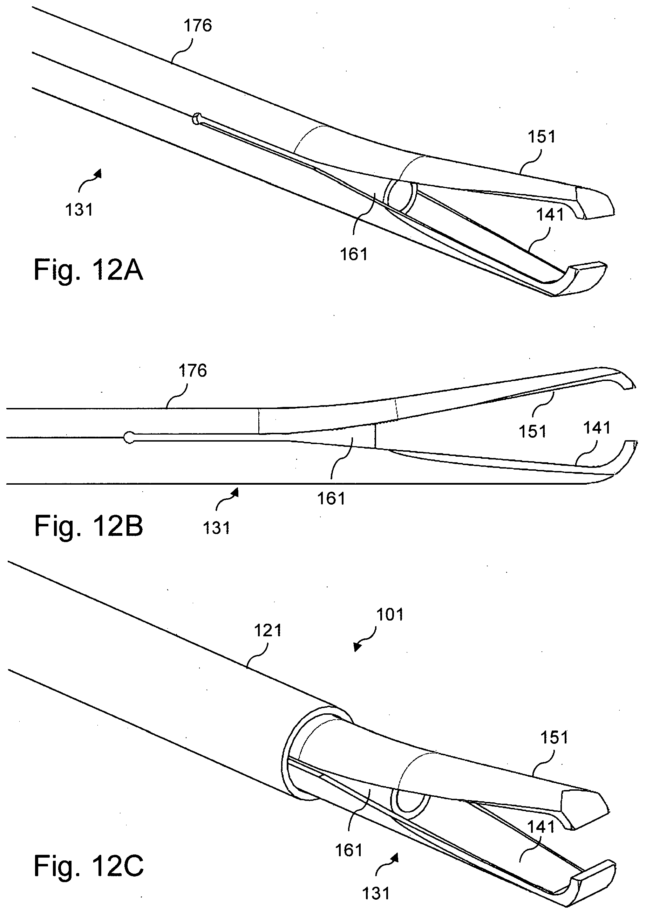

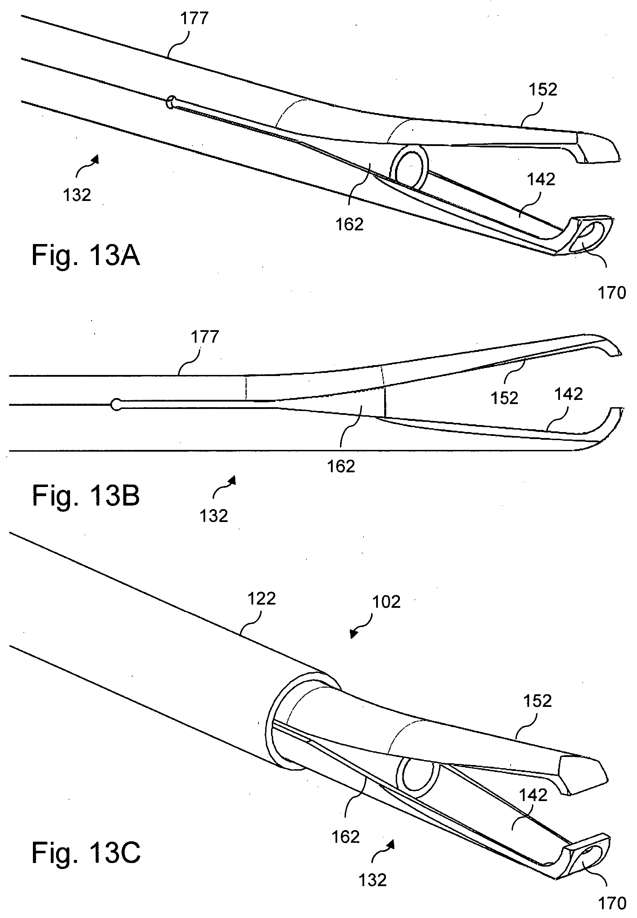

**Figs. 12A-12C** show a distal part of a surgical device 101 for use in intraocular surgery. The surgical device 101 includes an outer tube 121, an instrument insert 131, and an optical fiber 161. The instrument insert 131 may extend distally from the outer tube 121 and include two working ends, namely a first working end 141, functioning as a fixed jaw, and a second working end 151, functioning as a movable jaw. The instrument insert 131 may be formed from a single inner tube 176, with the jaws 141 and 151 manufactured directly from its distal end. The manufacturing process may for example involve wire electrical discharge machining (wire-EDM). The fixed jaw 141 may extend laterally from the longitudinal axis of the instrument insert 131 and remain substantially stationary during operation. The movable jaw 151 may be structurally biased to diverge laterally from the longitudinal axis when in the open state, as shown in Figs. 12A-12C. When the outer tube 121 is advanced distally over the instrument insert 131, the movable jaw 151 may be urged towards the fixed jaw 141, allowing the working ends to perform tasks such as gripping or holding tissue. This configuration may ensure that only the movable jaw 151 undergoes significant motion during operation, while the fixed jaw 141 provides a stable reference point. The optical fiber 161 may be positioned within the inner tube 176 and may extend longitudinally through the instrument insert 131. Although not directly shown in Figs. 12A-12C, the contact surfaces of the jaws 141 and 151, with which the jaws contact each other upon actuation, may be modified to maintain forward visibility from the optical fiber 161 even in the closed state of the jaws. In some embodiments, the contact surfaces may include a cut-out or opening to ensure that the optical fiber's line of sight remains unobstructed even when the jaws are in the closed state. An example thereof is shown in Figs. 13A-13C. Fig. 12A provides an isometric view of the instrument insert 131, showing the fixed jaw 141 and the movable jaw 151, with the movable jaw 151 in its open state. Fig. 12B shows a side view of the instrument insert 131, illustrating the structural bias of the movable jaw 151 to diverge from the longitudinal axis in the open state. Fig. 12C shows an isometric view of the distal end of the surgical device 101, where the instrument insert 131 and the optical fiber 161 emerge from the outer tube 121.

**Figs. 13A-13C** show a distal part of a surgical device 102 for use in intraocular surgery. The surgical device 102 includes an outer tube 122, an instrument insert 132, and an optical fiber 162. The instrument insert 132 comprises a fixed jaw 142 and a movable jaw 152, both formed from a single inner tube 177. The optical fiber 162 extends longitudinally through the inner tube 177. The fixed jaw 142 includes an opening 170 at its distal end, which is aligned with the distal end of the optical fiber 162. This opening 170 may ensure forward visibility from the optical fiber 162 even when the jaws 142 and 152 are in the closed state. The opening 170 may allow sensing signals from the optical fiber 162 to remain unobstructed, supporting intraocular sensing capabilities during operation. The details of the instrument insert 132 and its components, including the fixed jaw 142 and the movable jaw 152, may otherwise be similar to those described for Figs. 12A-12C, with the addition of the opening 170 in the fixed jaw 142. Fig. 13A provides an isometric view of the instrument insert 132, Fig. 13B shows a side view of the instrument insert 132, and Fig. 13C shows an isometric view of the distal end of the surgical device 102.

In general, the presently disclosed surgical devices may be manufactured using various techniques. In some embodiments, wire-EDM may be used to form the working ends, lumens, and other intricate geometries. A single thick-walled tube may be machined to form both the inner tube and the instrument insert as an integral structure. The inner tube may be formed by reducing its diameter along a longitudinal portion, while the working ends may be machined in an open state or in a closed state before being biased to the open position. Wire-EDM may also be used to create asymmetric or wedge-shaped designs, as well as diverging jaws and lumens for optical fiber integration. Welding may be used to assemble components. Movable jaws may be separately manufactured and welded to the inner tube or an intermediate tube arranged between the inner and outer tubes. Alternatively, the instrument insert may be welded to an inner rod that includes a groove or lumen to house the optical fiber. Off-the-shelf forceps may also be incorporated into the device, for example by welding the forceps to an inner rod or by using the jaws of the forceps as movable jaws. Multi-lumen guides, which may include grooves or lumens for the optical fiber and instrument insert, may be fabricated and integrated to simplify component alignment and stabilization. These approaches enable a compact and precise profile suitable for minimally invasive intraocular procedures, for example with a 23G or 25G profile.

It should be noted that the above-mentioned embodiments illustrate rather than limit the presently disclosed subject matter, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The mere fact that certain measures are recited in mutually different dependent claims does not indicate that a combination of these measures cannot be used to advantage.

## Claims

1. A surgical device (1-10) for use in microsurgery, comprising:
- an outer tube (20-29) comprising a distal end;
- an instrument insert (30-39) extending axially from the distal end of the outer tube, wherein the instrument insert comprises at least two working ends (40-59), wherein the outer tube is configured to slidably move (S) over the instrument insert, wherein the at least two working ends are structurally biased to an open state, wherein the instrument insert is actuated by the outer tube being advanced distally over the instrument insert to cause the outer tube to urge the at least two working ends to a closed state; and
- an optical fiber (60-69) for intraoperative sensing, wherein the optical fiber is arranged relative to the instrument insert to have axial forward visibility when the at least two working ends are in the closed state.

2. The surgical device (1-3) according to claim 1, wherein the instrument insert (30-32) comprises a junction (73) from which the at least two working ends (40-42, 50-52) originate, wherein the optical fiber (60-62) extends longitudinally through the instrument insert (30-32) and projects outwardly from the junction.

3. The surgical device (1-3) according to claim 2, wherein the optical fiber (60-62) is concentrically aligned with the instrument insert (30-32) at the junction (73).

4. The surgical device (1, 3) according to claim 2 or 3, wherein the at least two working ends (40, 42, 50, 52) comprise an opening (70, 71) that is axially aligned with the distal end of the optical fiber (60, 62) when the at least two working ends are in the closed state.

5. The surgical device (2) according to claim 2 or 3, wherein the at least two working ends (41, 51) are configured to diverge laterally from a longitudinal axis (L) of the instrument insert (31) as they extend distally from the junction, creating an unobstructed axial path for the optical fiber.

6. The surgical device (1-6) according to any one of claims 1 to 5, wherein the outer tube comprises an inner tube (75-77), wherein the instrument insert (30-35) is formed from a distal end of the inner tube to form an integral structure with the inner tube.

7. The surgical device (1-6) according to claim 6, wherein the at least two working ends (40-45, 50-55) are formed from the distal end of the inner tube using electrical discharge machining (EDM).

8. The surgical device (1-6) according to claim 6 or 7, wherein the inner tube (75-77) and the instrument insert (30-35) are manufactured from a single thick-walled tube, wherein the inner tube is formed by circumferentially reducing a diameter of a longitudinal portion of the thick-walled tube, wherein the instrument insert is formed in its open state from the thick-walled tube.

9. The surgical device (1-6) according to claim 8, wherein the inner tube (70-75) and/or the instrument insert (30-35) are formed from the single thick-walled tube using EDM.

10. The surgical device (4-6) according to any one of claims 1 to 9, wherein the at least two working ends comprise:
- a first working end (43-45) functioning as a substantially fixed working end, wherein the first working end extends laterally from a longitudinal axis of the outer tube (23-25) and maintains its position and orientation in the open state and the closed state; and
- a second working end (53-55) functioning as a movable working end, wherein the second working end diverges laterally from the longitudinal axis of the outer tube in the open state, wherein the second working end is urged towards the first working end when the outer tube is advanced distally over the instrument insert.

11. The surgical device (4-6) according to claim 10, wherein the first working end (43-45). comprises a lumen (78, 79), wherein the optical fiber extends (63-65) longitudinally through the lumen.

12. The surgical device (4-6) according to claim 10 or 11, further comprising an inner tube arranged in the outer tube, wherein one of the first working end (43-45) and the second working end (53-55) is formed by the inner tube and another one of the first working end (43-45) and the second working end (53-55) is welded to the inner tube or formed by an intermediate tube (80) arranged between the inner tube and the outer tube (25).

13. The surgical device (7-10) according to claim 1, wherein the outer tube (26-29) comprises an inner rod (83, 85, 86), wherein the instrument insert (36-39) is axially welded to a distal end of the inner rod, wherein the optical fiber (66-69) is arranged off-axially with respect to the instrument insert in a groove (84) or lumen (89) of the inner rod and projects outwardly from the distal end of the inner rod.

14. The surgical device (7-10) according to claim 13, wherein the instrument insert is eccentrically welded to the distal end of the inner rod (83, 85, 86), wherein the inner rod is arranged eccentrically within the outer tube (26-29) so that the at least two working ends (46-49, 56-59) are centrically positioned with respect to a longitudinal axis of the surgical device.

15. A method of manufacturing a surgical device (1-10), comprising:
- providing an outer tube (20-29) comprising a distal end;
- providing an instrument insert (30-39) which comprises at least two working ends, wherein the at least two working ends are structurally biased to an open state,
- mutually arranging the outer tube and the instrument insert so that the instrument insert extends axially from the distal end of the outer tube and the outer tube is slidably movable (S) over the instrument insert to enable actuation of the instrument insert when the outer tube is advanced distally over the instrument insert to cause the outer tube to urge the at least two working ends to a closed state;
- providing an optical fiber (60-69) for intraoperative sensing;
- arranging the optical fiber relative to the instrument insert to have axial forward visibility when the at least two working ends are in the closed state.
